# EUROPEAN PATENT APPLICATION

(11) **EP 4 802 992 A1**
(43) Date of publication of application: **09.09.2026**
(21) Application number: 25755153.1
(22) Date of filing: 12.02.2025
(51) Int. Cl.: A61B 5/00, A61B 5/11, A61B 5/053, A61B 5/024

(54) **WEARABLE DEVICE FOR ESTIMATING AMOUNT OF SWEAT LOSS AND CONTROL METHOD THEREFOR**

(30) Priority: 13.02.2024 KR 20240020258; 08.04.2024 KR 20240047393
(71) Applicant: Samsung Electronics Co., Ltd., Suwon-si, Gyeonggi-do 16677 (KR)
(72) Inventor: OH, Youngjae, Suwon-si Gyeonggi-do 16677 (KR); MIN, Jinhong, Suwon-si Gyeonggi-do 16677 (KR); YOON, Seoyoung, Suwon-si Gyeonggi-do 16677 (KR); JEON, Taehan, Suwon-si Gyeonggi-do 16677 (KR); CHOI, Hyoungseon, Suwon-si Gyeonggi-do 16677 (KR); JUNG, Jiwoon, Suwon-si Gyeonggi-do 16677 (KR); KIM, Taeseon, Suwon-si Gyeonggi-do 16677 (KR)
(74) Representative: Gulde & Partner
(86) International application number: PCT/KR2025/002059
(87) International publication number: WO 2025/174055

(57) **Abstract**

Provided is a wearable device for estimating sweat loss. The wearable device comprises: at least one first sensor; at least one second sensor including a plurality of electrodes; a memory for storing one or more computer programs; and at least one processor communicatively coupled to the at least one first sensor, the at least one second sensor, and the memory. The one or more computer programs include computer-executable instructions that, when individually or collectively executed by the at least one processor, cause the wearable device to: use the at least one first sensor to identify whether a user wearing the wearable device starts exercise; identify a point in time of perspiration of the user by using the at least one second sensor according to the identification that the user starts exercise; and determine the level of perspiration of the user on the basis of the identified point in time of perspiration.

## Description

### [TECHNICAL FIELD]

The disclosure relates to a wearable device estimating a sweat loss and a method for controlling the same.

### [BACKGROUND ART]

More and more services and additional functions are being provided through wearable devices, e.g., smart watches, or other portable electronic devices. To meet the needs of various users and raise use efficiency of electronic devices, communication service carriers or device manufacturers are jumping into competitions to develop electronic devices with differentiated and diversified functionalities. Accordingly, various functions that are provided through wearable devices are evolving more and more.

The above information is presented as background information only to assist with an understanding of the disclosure. No determination has been made, and no assertion is made, as to whether any of the above might be applicable as prior art with regard to the disclosure.

### [DISCLOSURE OF INVENTION]

### [Solution to Problems]

The related art provides only information about the average sweat loss based only on basic information, such as the user's gender, age, or weight, but may not provide information about the sweat loss considering the user's sweating characteristics.

Aspects of the disclosure are to address at least the above-mentioned problems and/or disadvantages and to provide at least the advantages described below. Accordingly, an aspect of the disclosure is to provide a wearable device estimating a sweat loss and a method for controlling the same.

Additional aspects will be set forth in part in the description which follows and, in part, will be apparent from the description, or may be learned by practice of the presented embodiments.

In accordance with an aspect of the disclosure, a wearable device is provided. The wearable device includes at least one first sensor, at least one second sensor including a plurality of electrodes, memory storing one or more computer programs, and one or more processors communicatively coupled to the at least one first sensor, the at least one second sensor, and the memory, wherein the one or more computer programs include computer-executable instructions that, when executed by the one or more processors, cause the wearable device to identify whether, using the at least one first sensor, a user wearing the wearable device starts exercise, based on the identifying that the user starts the exercise, identify, using the at least one second sensor, a time of sweating of the user, and based on the identified time of sweating, determine a degree of sweat loss of the user.

In accordance with another aspect of the disclosure, a method performed by a wearable device, the wearable device including at least one first sensor and at least one second sensor including a plurality of electrodes is provided. The method includes identifying, by the wearable device, whether, using the at least one first sensor, a user wearing the wearable device starts exercise, based on the identifying that the user starts the exercise, identifying, by the wearable device, using the at least one second sensor, a time of sweating of the user, and based on the identified time of sweating, determining, by the wearable device, a degree of sweat loss of the user.

In accordance with another aspect of the disclosure, one or more non-transitory computer-readable storage media storing one or more computer programs including computer-executable instructions that, when executed by one or more processors of a wearable device individually or collectively, the wearable device including at least one first sensor and at least one second sensor cause the wearable device to perform operations are provided. The operations include identifying, by the wearable device, whether, using the at least one first sensor, a user wearing the wearable device starts exercise, based on the identifying that the user starts the exercise, identifying, by the wearable device, using the at least one second sensor, a time of sweating of the user, and based on the identified time of sweating, determining, by the wearable device, a degree of sweat loss of the user.

According to various embodiments of the disclosure, there may be provided a wearable device and a method for controlling the same, which may provide information about sweat loss considering even the sweating characteristics of the user to thereby provide relatively accurate sweat loss information to the user.

Other aspects, advantages, and salient features of the disclosure will become apparent to those skilled in the art from the following detailed description, which, taken in conjunction with the annexed drawings, discloses various embodiments of the disclosure.

### [BRIEF DESCRIPTION OF DRAWINGS]

The above and other aspects, features, and advantages of certain embodiments of the disclosure will be more apparent from the following description taken in conjunction with the accompanying drawings, in which:
FIG. 1 illustrates an electronic device (e.g., a wearable device) according to an embodiment of the disclosure;
FIG. 2A is a front perspective view illustrating a wearable device according to an embodiment of the disclosure;
FIG. 2B is a rear perspective view illustrating a wearable electronic device of FIG. 2A according to an embodiment of the disclosure;
FIG. 3 is an exploded perspective view illustrating an electronic device of FIG. 2A according to an embodiment of the disclosure;
FIG. 4 illustrates a function or operation of determining (e.g., estimating) a sweat loss by a wearable device according to an embodiment of the disclosure;
FIG. 5A illustrates a function or operation of determining or estimating a time of sweating according to a change in impedance by a wearable device according to an embodiment of the disclosure;
FIG. 5B illustrates a function or operation of determining or estimating a time of sweating according to a change in phase value by a wearable device according to an embodiment of the disclosure;
FIG. 6A illustrates how a sweat loss changes based on a sweating characteristic of a user according to an embodiment of the disclosure;
FIG. 6B illustrates how a sweat loss changes based on an exercise intensity of a user according to an embodiment of the disclosure;
FIG. 7A illustrates a function or operation of estimating a sweat loss and providing an estimated sweat loss to a user according to the related art; and
FIG. 7B illustrates a function or operation of estimating a sweat loss based on a sweating characteristic of a user and providing an estimated sweat loss to a user according to an embodiment of the disclosure.

The same reference numerals are used to represent the same elements throughout the drawings.

### [MODE FOR THE INVENTION]

The following description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

It should be appreciated that the blocks in each flowchart and combinations of the flowcharts may be performed by one or more computer programs which include computer-executable instructions. The entirety of the one or more computer programs may be stored in a single memory device or the one or more computer programs may be divided with different portions stored in different multiple memory devices.

Any of the functions or operations described herein can be processed by one processor or a combination of processors. The one processor or the combination of processors is circuitry performing processing and includes circuitry like an application processor (AP, e.g., a central processing unit (CPU)), a communication processor (CP, e.g., a modem), a graphical processing unit (GPU), a neural processing unit (NPU) (e.g., an artificial intelligence (AI) chip), a wireless-fidelity (Wi-Fi) chip, a Bluetooth^{™} chip, a global positioning system (GPS) chip, a near field communication (NFC) chip, connectivity chips, a sensor controller, a touch controller, a finger-print sensor controller, a display drive integrated circuit (IC), an audio CODEC chip, a universal serial bus (USB) controller, a camera controller, an image processing IC, a microprocessor unit (MPU), a system on chip (SoC), an IC, or the like.

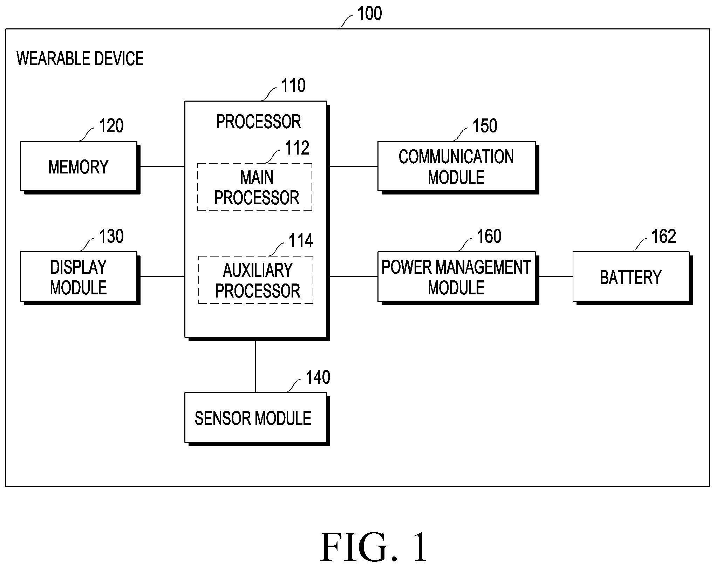

FIG. 1 illustrates an electronic device according to an embodiment of the disclosure.

Referring to FIG. 1, a wearable device 100 according to an embodiment of the disclosure may include a processor 110, memory 120, a display module 130, a sensor module 140, a communication module 150, a power management module 160, and a battery 162.

The processor 110 according to an embodiment of the disclosure may execute, e.g., software to control at least one other component (e.g., a hardware or software component) of the wearable device 100 connected with the processor 110 and may process or compute various data. According to an embodiment of the disclosure, as at least part of the data processing or computation, the processor 110 may store a command or data received from another component (e.g., the sensor module 140 or communication module 170 or a sensor module 140) onto a volatile memory, process the command or the data stored in the volatile memory, and store resulting data in the memory 120 (e.g., non-volatile memory). According to an embodiment of the disclosure, the processor 110 may include a main processor 112 (e.g., a central processing unit (CPU) or an application processor (AP)), or an auxiliary processor 114 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the wearable device 100 includes the main processor 112 and the auxiliary processor 114, the auxiliary processor 114 may be configured to use lower power than the main processor 112 or to be specified for a designated function. The auxiliary processor 114 may be implemented as separate from, or as part of the main processor 112.

The auxiliary processor 114 may control at least some of functions or states related to at least one component (e.g., the display module 130, the sensor module 140, or the communication module 150) among the components of the wearable device 100, instead of the main processor 112 while the main processor 112 is in an inactive (e.g., a sleep) state, or together with the main processor 112 while the main processor 112 is in an active state (e.g., executing an application). According to an embodiment of the disclosure, the auxiliary processor 114 (e.g., an image signal processor or a communication processor) may be implemented as part of another component (e.g., the camera module or the communication module 150) functionally related to the auxiliary processor 123. According to an embodiment of the disclosure, the auxiliary processor 123 (e.g., the neural processing unit) may include a hardware structure specified for artificial intelligence model processing. The artificial intelligence model may be generated via machine learning. Such learning may be performed, e.g., by the wearable device 100 where the artificial intelligence model is performed or via a separate server. Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

According to an embodiment of the disclosure, the memory 120 may store various data used by at least one component (e.g., the processor 110 or the sensor module 140) of the wearable device 100. The data may include, e.g., software (e.g., a program) and input data or output data for a command related thereto. The memory 120 may include volatile memory or nonvolatile memory.

According to an embodiment of the disclosure, the display module 130 may visually provide information to the outside (e.g., the user) of the wearable device 100. The display module 130 may include, for example, a display, a hologram device, or a projector and control circuitry to control a corresponding one of the display, hologram device, and projector. According to an embodiment of the disclosure, the display module 130 may include a touch sensor configured to detect a touch, or a pressure sensor configured to measure the intensity of a force generated by the touch.

The sensor module 140 according to an embodiment of the disclosure may detect an operational state (e.g., power or temperature) of the wearable device 100 or an environmental state (e.g., a state of a user) external to an electronic device 101, and then generate an electrical signal or data value corresponding to the detected state. According to an embodiment of the disclosure, the sensor module 140 may include, for example, a gesture sensor, a gyro sensor, an atmospheric pressure sensor, a magnetic sensor, an accelerometer, a grip sensor, a proximity sensor, a color sensor, an infrared (IR) sensor, a biometric sensor, a temperature sensor, a humidity sensor, a photoplethysmography (PPG) sensor, or an illuminance sensor. The sensor module 140 according to an embodiment of the disclosure may include a plurality of electrodes. The plurality of electrodes according to an embodiment of the disclosure may contact the user's skin while being worn on the user. The plurality of electrodes according to an embodiment of the disclosure may be electrically connected to each other.

The communication module 150 according to an embodiment of the disclosure may establish a direct (e.g., wired) communication channel or a wireless communication channel between the wearable device 100 and the external electronic device (e.g., a server) or support communication through the established communication channel. The communication module 150 may include one or more communication processors that are operable independently from the processor 110 (e.g., the application processor (AP)) and supports a direct (e.g., wired) communication or a wireless communication. According to an embodiment of the disclosure, the communication module 150 may include a wireless communication module (e.g., a cellular communication module, a short-range wireless communication module, or a global navigation satellite system (GNSS) communication module) or a wired communication module (e.g., a local area network (LAN) communication module or a power line communication (PLC) module). The communication module according to an embodiment of the disclosure may communicate with the external electronic device via a first network (e.g., a short-range communication network, such as Bluetooth^{™}, wireless-fidelity (Wi-Fi) direct, or infrared data association (IrDA)) or a second network (e.g., a long-range communication network, such as a legacy cellular network, a fifth generation (5G) network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module may identify or authenticate the wearable device 100 in a communication network, such as the first network or the second network, using subscriber information (e.g., international mobile subscriber identity (IMSI)) stored in the subscriber identification module.

The wireless communication module may support a 5G network, after a fourth generation (4G) network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the millimeter wave (mmWave) band) to achieve, e.g., a high data transmission rate. The wireless communication module may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module may support various requirements specified in the wearable device 100, an external electronic device, or a network system (e.g., the second network). According to an embodiment of the disclosure, the wireless communication module may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The power management module 160 according to an embodiment of the disclosure may manage power supplied to the wearable device 100. According to an embodiment of the disclosure, the power management module 160 may be implemented as at least part of, for example, a power management integrated circuit (PMIC).

The battery 162 according to an embodiment of the disclosure may supply power to at least one component of the wearable device 100. According to an embodiment of the disclosure, the battery 162 may include, for example, a primary cell which is not rechargeable, a secondary cell which is rechargeable, or a fuel cell.

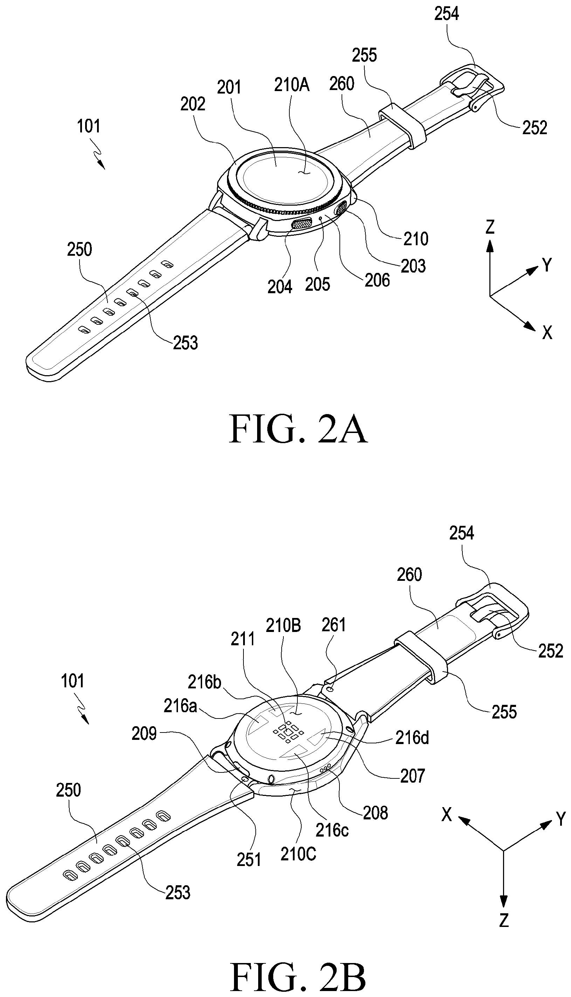

FIG. 2A is a front perspective view illustrating a wearable device according to an embodiment of the disclosure. FIG. 2B is a rear perspective view illustrating a wearable electronic device of FIG. 2A according to an embodiment of the disclosure. The embodiment(s) of FIGS. 2A and 2B may be selectively combined with the embodiment of FIG. 1 or the embodiment(s) described below to implement additional embodiments.

Referring to FIGS. 2A and 2B, a wearable device 100 may include a housing 210 or wearing members 250 and 260. The housing 210 may include a first surface (or front surface) 210A, a second surface (or rear surface) 210B, or a side surface 210C. The side surface 210C may surround a space between the first surface 210A and the second surface 210B. In an embodiment of the disclosure, the wearing members 250 and 260 may be connected to at least a portion of the housing 210 and be configured to detachably fasten the wearable device 100 to the user's body portion (e.g., wrist or ankle). For example, the wearable device 100 may be of a wrist watch type.

According to an embodiment of the disclosure, the housing 210 may denote a structure forming the first surface 210A of FIG. 2A, the second surface 210B of FIG. 2B, and some of the side surfaces 210C. In an embodiment of the disclosure, at least part of the first surface 210A may have a substantially transparent front plate 201 (e.g., a glass plate or polymer plate including various coat layers). The second surface 210B may be formed by a rear plate 207 that is substantially opaque. According to an embodiment of the disclosure, when the wearable device includes a sensor module 211 disposed on the second surface 210B, the rear plate 207 may at least partially include a transparent area. The rear plate 207 may be formed of, e.g., laminated or colored glass, ceramic, polymer, metal (e.g., aluminum, stainless steel (STS), or magnesium), or a combination of at least two thereof. The side surface 210C may be formed by a side bezel structure (or a "side member") 206 that couples to the front plate 201 and the rear plate 207 and includes a metal and/or polymer. According to an embodiment of the disclosure, the rear plate 207 and the side bezel plate 206 may be integrally formed together and include the same material (e.g., a metal, such as aluminum). The wearing members 250 and 260 may be formed of various materials in various shapes. A uni-body structure or multiple unit links which is flexible may be formed of fabric, leather, rubber, urethane, metal, ceramic, or a combination of at least two thereof.

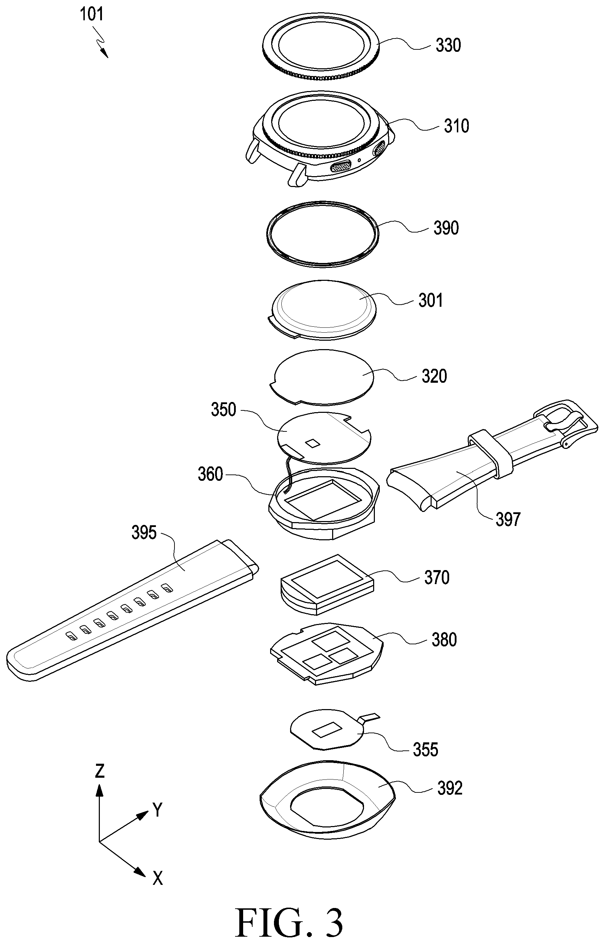

According to an embodiment of the disclosure, the wearable device 100 may include at least one or more of a display (e.g., a display 320 of FIG. 3), audio modules 205 and 208, a sensor module 211 (e.g., the sensor module 140 of FIG. 1), key input devices 202, 203, and 204, or a connector hole 209. According to an embodiment of the disclosure, the wearable device 100 may exclude at least one (e.g., the key input devices 202, 203, and 204, connector hole 209, or sensor module 211) of the components or may add other components.

According to an embodiment of the disclosure, the display (e.g., the display 320 of FIG. 3) may be visually exposed through a significant portion of the front plate 201. The display may have a shape corresponding to the shape of the front plate 201, e.g., a circle, ellipse, or polygon. The display may be coupled with, or disposed adjacent, a touch detection circuit, a pressure sensor capable of measuring the strength (pressure) of touches, and/or fingerprint sensor.

The audio modules 205 and 208 may include a microphone hole 205 and a speaker hole 208. A microphone for acquiring external sounds may be disposed in the microphone hole 205. In an embodiment of the disclosure, a plurality of microphones may be disposed to detect the direction of the sound. The speaker hole 208 may be used for an external speaker. According to an embodiment of the disclosure, a speaker may be included without the speaker hole (e.g., a piezo speaker).

The sensor module 211 may produce an electrical signal or data value corresponding to the internal operation state or external environment state of the wearable device 100. The sensor module 211 may include, e.g., a biometric sensor module 211 (e.g., a heartrate monitor (HRM) sensor) disposed on the second surface 210B of the housing 210. The wearable device 100 may include a sensor module not shown, e.g., at least one of a gesture sensor, a gyro sensor, a barometric sensor, a magnetic sensor, an acceleration sensor, a grip sensor, a color sensor, an infrared (IR) sensor, a biometric sensor, a temperature sensor, a humidity sensor, or an illuminance sensor.

The key input devices 202, 203, and 204 may include a wheel key 202 disposed on the first surface 210A of the housing 210 to be rotatable in at least one direction and/or key buttons 203 and 204 disposed on the side surface 210C of the housing 210. The wheel key 202 may have a shape corresponding to the shape of the front plate 201. According to an embodiment of the disclosure, the wearable device 100 may exclude all or some of the above-mentioned key input devices 202, 203, and 204 and the excluded key input devices 202, 203, and 204 may be implemented in other forms, e.g., as soft keys on the display. The connector hole 209 may receive a connector (e.g., a universal serial bus (USB) connector) for transmitting and receiving power and/or data to/from an external electronic device. Another connector hole (not shown) may be included for receiving a connector for transmitting and receiving audio signals to/from the external electronic device. The wearable device 100 may further include a connector cover (not shown) to cover at least part of, e.g., the connector hole 209 and preventing undesirable materials from entering the connector hole.

The wearing members 250 and 260 may detachably be fastened to at least portions of the housing 210 via locking members 251 and 261. The locking members 251 and 261 may include components or parts for coupling, such as pogo pins, and, according to an embodiment of the disclosure, may be replaced with protrusions or recesses formed on/in the wearing members 250 and 260. For example, the wearing members 250 and 260 may be coupled in such a manner as to be fitted into or over the recesses or protrusions formed on the housing 210. The wearing members 250 and 260 may include one or more of a fastening member 252, fastening member coupling holes 253, a band guide member 254, and a band fastening ring 255.

The fastening member 252 may be configured to allow the housing 210 and the wearing members 250 and 260 to be fastened to the user's body portion (e.g., wrist or ankle). The fastening member coupling holes 253 may fasten the housing 210 and the wearing members 250 and 260 to the user's body portion, corresponding to the fastening member 252. The band guide member 254 may be configured to restrict movement of the fastening member 252 to a certain range when the fastening member 252 fits into one of the fastening member coupling holes 253, thereby allowing the wearing members 250 and 260 to be tightly fastened onto the user's body portion. The band fastening ring 255 may limit the range of movement of the wearing members 250 and 260, with the fastening member 252 fitted into one of the fastening member coupling holes 253.

FIG. 3 is an exploded perspective view illustrating an electronic device of FIG. 2A according to an embodiment of the disclosure. The embodiment of FIG. 3 may be selectively combined with the embodiment(s) of FIGS. 1, 2A, and 2B, or the embodiment(s) described below to implement additional embodiments.

Referring to FIG. 3, a wearable device 100 (e.g., the wearable device 100 of FIG. 1 or the wearable device 100 of FIGS. 2A and 2B) may include a side bezel structure 310 (e.g., the side bezel structure 206 of FIGS. 2A and 2B), a wheel key 330 (e.g., the wheel key 202 of FIGS. 2A and 2B), a front plate 301 (e.g., the front plate 201 of FIG. 2A), a display 320, a first antenna 350, a second antenna (e.g., the antenna included in the second circuit board 355), a supporting member 360 (e.g., a bracket), a battery 370, a printed circuit board 380, a sealing member 390, a rear plate 392, and wearing members 395 and 397 (e.g., the wearing members 250 and 260 of FIG. 2A or 2B). At least one of the components of the wearable device 100 may be the same or similar to at least one of the components of the wearable device 100 of FIG. 2A or 2B and no duplicate description is made below.

According to an embodiment of the disclosure, the supporting member 360 may be disposed inside the wearable device 100 to be connected with the side bezel structure 310 (e.g., a side member) or integrated with the side bezel structure 310. The supporting member 360 may be formed of, e.g., a metal and/or non-metallic material (e.g., polymer). The display 320 (e.g., the display module 130 of FIG. 1) may be joined onto one surface of the supporting member 360, and the printed circuit board 380 may be joined onto the opposite surface of the supporting member 274. A processor, memory, and/or interface may be mounted on the printed circuit board 380.

The processor (e.g., the processor 110 of FIG. 1) may include one or more of, e.g., a central processing unit, an application processor, a graphic processing unit (GPU), a sensor processor, or a communication processor.

The memory (e.g., the memory 120 of FIG. 1) may include, e.g., volatile or non-volatile memory.

The interface may include, e.g., a high definition multimedia interface (HDMI), a universal serial bus (USB) interface, a secure digital (SD) card interface, and/or an audio interface. The interface may electrically or physically connect, e.g., the wearable device 100 with an external electronic device and may include a USB connector, an SD card/multimedia card (MMC) connector, or an audio connector.

According to an embodiment of the disclosure, the battery 370 (e.g., the battery 162 of FIG. 1) may be a device for supplying power to at least one component of the wearable device 100. The battery 370 may include, e.g., a primary cell which is not rechargeable, a secondary cell which is rechargeable, or a fuel cell. At least a portion of the battery 370 may be disposed on substantially the same plane as the printed circuit board 380. The battery 370 may be integrally or detachably disposed inside the wearable device 100.

According to an embodiment of the disclosure, the first antenna 350 may be disposed between the display 320 and the supporting member 360. The first antenna 350 may include, e.g., a near-field communication (NFC) antenna, a wireless charging antenna, and/or a magnetic secure transmission (MST) antenna. The first antenna 350 may perform short-range communication with an external device, wirelessly transmit/receive power necessary for charging, or transmit magnetic-based signals including payment data or short-range communication signals. In an embodiment of the disclosure, an antenna structure may be formed by a portion or combination of the side bezel structure 310 and/or the supporting member 360.

According to an embodiment of the disclosure, the second circuit board 355 may be disposed between the circuit board 380 and the rear plate 392. The second circuit board 355 may include an antenna (e.g., the antenna module 197 of FIG. 1), e.g., a near-field communication (NFC) antenna, a wireless charging antenna, and/or a magnetic secure transmission (MST) antenna. For example, the second circuit board 355 may perform short-range communication with an external device, wirelessly transmit/receive power necessary for charging, or transmit magnetic-based signals including payment data or short-range communication signals. According to an embodiment of the disclosure, an antenna structure may be formed of a portion or combination of the side bezel structure 310 and/or the rear plate 392. According to various embodiments of the disclosure, when the wearable device 100 (e.g., the wearable device 100 of FIGS. 2A and 2B) includes a sensor module (e.g., the sensor module 211 of FIG. 2B), the sensor circuit disposed on the second circuit board 355 or a sensor element (e.g., a photoelectric conversion element or an electrode pad) separate from the second circuit board 355 may be disposed. For example, an electronic component provided as the sensor module may be disposed between the circuit board 380 and the rear plate 392.

According to an embodiment of the disclosure, the sealing member 390 may be positioned between the side bezel structure 310 and the front plate 301. The sealing member 390 may be configured to block or reduce moisture or foreign bodies that may enter the space surrounded by the side bezel structure 310 and the front plate 301, from the outside.

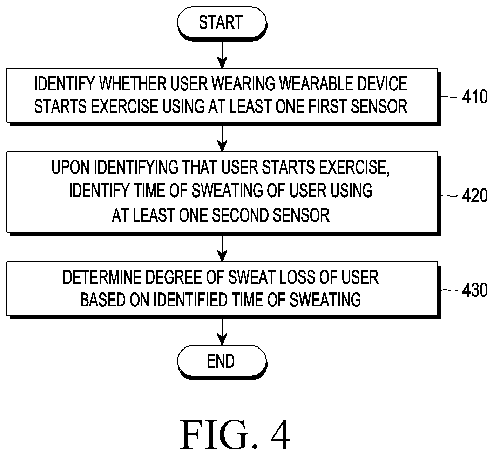

FIG. 4 illustrates a function or operation of determining (e.g., estimating) a sweat loss by a wearable device according to an embodiment of the disclosure.

Referring to FIG. 4, in operation 410, the wearable device 100 according to an embodiment of the disclosure may identify whether a user wearing the wearable device 100 starts exercise using at least one first sensor (e.g., a PPG sensor, a temperature sensor, and/or an acceleration sensor). The wearable device 100 according to an embodiment of the disclosure may compare sensing data detected by the acceleration sensor with sample data to determine whether the user is currently exercising and/or what kind of exercise the user is performing. The wearable device 100 according to an embodiment of the disclosure may determine whether the user is currently exercising, based on whether the user's heart rate is larger than or equal to a designated threshold and/or whether the user maintains the threshold or more for a designated time in addition to the data detected by the acceleration sensor. For example, when the heart rate of the user determined based on the data detected by the PPG sensor maintains a designated threshold or more for the designated time or more, the wearable device 100 according to an embodiment of the disclosure may determine that the user is currently exercising, even if the sensing data obtained by the acceleration sensor does not indicate the user's exercise. For example, when the skin temperature of the user determined based on the data detected by the temperature sensor configured to detect the skin temperature maintains a designated threshold or more for a designated time or more, the wearable device 100 according to an embodiment of the disclosure may determine that the user is currently exercising, even if the sensing data obtained by the acceleration sensor does not indicate the user's exercise.

In operation 420, when it is identified that the user has started the exercise according to operation 410, the wearable device 100 according to an embodiment of the disclosure may determine the time of sweating of the user using the at least one second sensor. The second sensor (e.g., the sensor module 140 of FIG. 1) according to an embodiment of the disclosure may include a plurality of electrodes (e.g., the first electrode 216a, the second electrode 216b, the third electrode 216c, and/or a fourth electrode 216d). The plurality of electrodes (e.g., the first electrode 216a, the second electrode 216b, the third electrode 216c, and/or the fourth electrode 216d) according to an embodiment of the disclosure may be electrically connected to each other. The processor 110 according to an embodiment of the disclosure may control the wearable device 100 to apply a current through the plurality of electrodes (e.g., the first electrode 216a, the second electrode 216b, the third electrode 216c, and/or the fourth electrode 216d) in a state of contacting the skin.

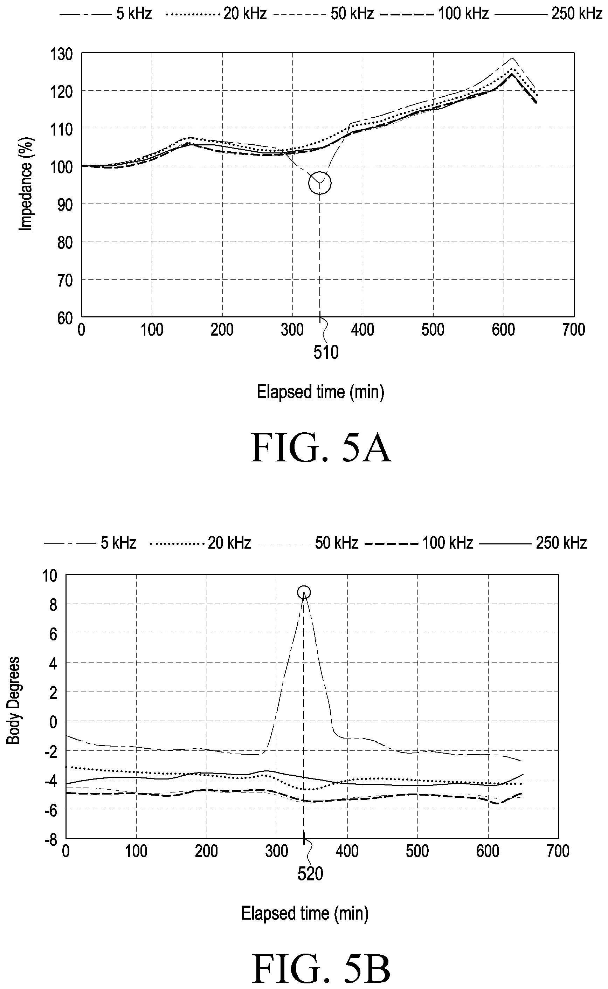

FIG. 5A illustrates a function or operation of determining or estimating a time of sweating according to a change in impedance by a wearable device according to an embodiment of the disclosure. FIG. 5B illustrates a function or operation of determining or estimating a time of sweating according to a change in phase value by a wearable device according to an embodiment of the disclosure.

Referring to FIG. 5A, the wearable device 100 according to an embodiment of the disclosure may apply an alternating current based on a designated frequency (e.g., a frequency of 5 kHz) to the plurality of electrodes (e.g., the first electrode 216a, the second electrode 216b, the third electrode 216c, and/or the fourth electrode 216d). The wearable device 100 according to an embodiment of the disclosure may identify a time (e.g., a first time 510) when impedance for the plurality of electrodes (e.g., the first electrode 216a, the second electrode 216b, the third electrode 216c, and/or the fourth electrode 216d) drops. The wearable device 100 according to an embodiment of the disclosure may determine or estimate the time (e.g., the first time 510) when the impedance for the plurality of electrodes (e.g., the first electrode 216a, the second electrode 216b, the third electrode 216c, and/or the fourth electrode 216d) drops as the time of sweating. The wearable device 100 according to an embodiment of the disclosure may apply an alternating current based on a plurality of frequencies to the plurality of electrodes (e.g., the first electrode 216a, the second electrode 216b, the third electrode 216c, and/or the fourth electrode 216d).

Referring to FIG. 5B, the wearable device 100 according to an embodiment of the disclosure may apply the alternating current based on the designated frequency (e.g., a frequency of 5 kHz) to the plurality of electrodes (e.g., the first electrode 216a, the second electrode 216b, the third electrode 216c, and/or the fourth electrode 216d). The wearable device 100 according to an embodiment of the disclosure may identify a time (e.g., a second time 520) when a phase value (e.g., an angle) for the plurality of electrodes (e.g., the first electrode 216a, the second electrode 216b, the third electrode 216c, and/or the fourth electrode 216d) is changed. The wearable device 100 according to an embodiment of the disclosure may determine or estimate the time (e.g., the second time 520) when the phase value for the plurality of electrodes (e.g., the first electrode 216a, the second electrode 216b, the third electrode 216c, and/or the fourth electrode 216d) is changed as the time of sweating. The wearable device 100 according to an embodiment of the disclosure may apply an alternating current based on a plurality of frequencies to the plurality of electrodes (e.g., the first electrode 216a, the second electrode 216b, the third electrode 216c, and/or the fourth electrode 216d).

The wearable device 100 according to an embodiment of the disclosure may identify both the time (e.g., the first time 510) when the impedance for the plurality of electrodes (e.g., the first electrode 216a, the second electrode 216b, the third electrode 216c, and/or the fourth electrode 216d) drops and the time (e.g., the second time 520) when the phase value for the plurality of electrodes (e.g., the first electrode 216a, the second electrode 216b, the third electrode 216c, and/or the fourth electrode 216d) is changed and, when a time difference between the first time 510 and the second time 520 is included within a designated error range, determine one of the first time 510 or the second time 520 as the time of sweating. The wearable device 100 according to an embodiment of the disclosure may identify both the time (e.g., the first time 510) when the impedance for the plurality of electrodes (e.g., the first electrode 216a, the second electrode 216b, the third electrode 216c, and/or the fourth electrode 216d) drops and the time (e.g., the second time 520) when the phase value for the plurality of electrodes (e.g., the first electrode 216a, the second electrode 216b, the third electrode 216c, and/or the fourth electrode 216d) is changed and, when a time difference between the first time 510 and the second time 520 falls outside the designated error range, determine the first time 510 as the time of sweating. The wearable device 100 according to an embodiment of the disclosure may identify both the time (e.g., the first time 510) when the impedance for the plurality of electrodes (e.g., the first electrode 216a, the second electrode 216b, the third electrode 216c, and/or the fourth electrode 216d) drops and the time (e.g., the second time 520) when the phase value for the plurality of electrodes (e.g., the first electrode 216a, the second electrode 216b, the third electrode 216c, and/or the fourth electrode 216d) is changed and, when a time difference between the first time 510 and the second time 520 falls outside the designated error range, determine the first time 510 or the second time 520 as the time of sweating according to the user's setting.

In operation 430, the wearable device 100 according to an embodiment of the disclosure may determine (e.g., estimate) the degree of sweat loss of the user, based on the time of sweating identified according to operation 420. The wearable device 100 according to an embodiment of the disclosure may determine the current exercise intensity of the user, based on the data detected according to operation 410. For example, the wearable device 100 according to an embodiment of the disclosure may determine the exercise intensity of the user based on a lookup table in which a relationship between the exercise intensity and the sensing data is defined. For example, when the user's heart rate is 150 or more, the wearable device 100 according to an embodiment of the disclosure may determine the intensity of exercise as "strong". Or, when the heart rate of the user has a value between 120 (e.g., 120 or more) and 150 (e.g., less than 150), the exercise intensity may be determined as "medium". Or, when the heart rate of the user has a value between 100 and 120, the exercise intensity may be determined as "weak". However, such classifications are provided as an example, and various exercise intensities may be defined in the lookup table.

The wearable device 100 according to an embodiment of the disclosure may select (e.g., determine) a sub data set corresponding to the determined exercise intensity and basic information about the user, such as the user's gender, age, weight, and height from the sample dataset.

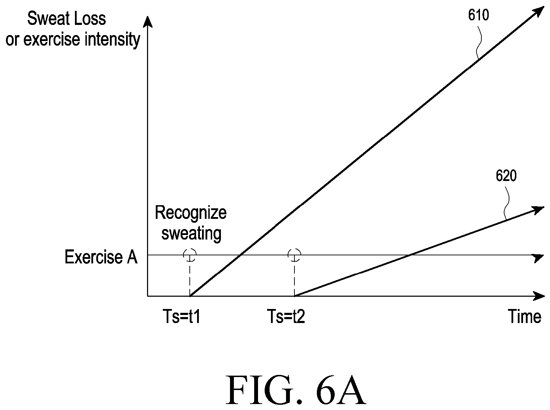

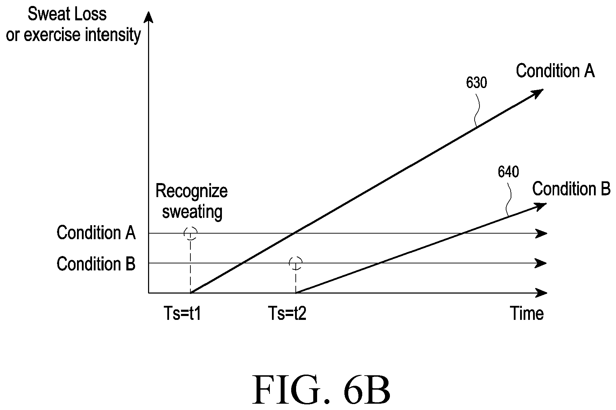

FIG. 6A illustrates how a sweat loss changes based on a sweating characteristic of a user according to an embodiment of the disclosure. FIG. 6B illustrates how a sweat loss changes based on an exercise intensity of a user according to an embodiment of the disclosure.

Referring to FIGS. 6A and 6B, the sample data 610, 620, 630, and 640 according to an embodiment of the disclosure may include, e.g., as illustrated in FIGS. 6A and 6B, straight-line data having various slopes depending on the exercise progress time and the sweat loss. The sample data according to an embodiment of the disclosure may be classified according to the exercise intensity and basic information about the user. The sample data according to an embodiment of the disclosure may be stored in the wearable device 100 or may be stored in an external device (e.g., a server) operably connected to the wearable device 100. As illustrated in FIGS. 6A and 6B, the sample data according to an embodiment of the disclosure may include data in the form of a straight line starting from various times of sweating (e.g., any specific point on the x-axis). For example, a specific straight line belonging to the sample data according to an embodiment of the disclosure may include data in the form of a straight line starting from (20,0) rather than the origin. As illustrated in FIG. 6A, the sample data and/or the sub data set according to an embodiment of the disclosure may include straight-line data having different slopes according to the time of sweating of the user. FIG. 6A illustrates a case in which different users having different sweating characteristics have different sweat losses. FIG. 6B illustrates a case in which even the same user has different sweat losses depending on the exercise intensity.

The wearable device 200 according to an embodiment of the disclosure may select at least one data (e.g., a straight line) corresponding to the time of sweating of the user from the selected sub data set. As illustrated in FIGS. 6A and 6B, the sub data set according to an embodiment of the disclosure may include straight-line data having different slopes according to the time of sweating. The wearable device 100 according to an embodiment of the disclosure may select at least one data (e.g., data having a time of sweating included within a first designated error range) having the time of sweating substantially corresponding to the time of sweating of the user in the sub data set as a coordinate value of the X-axis. When the sub data set does not have at least one data (e.g., data having a time of sweating included within the designated error range) substantially corresponding to the time of sweating of the user, the wearable device 100 according to an embodiment of the disclosure may select a plurality of data (e.g., data having a time of sweating included within a second designated error range having a wider error range than the first designated error range) having a time of sweating similar to the time of sweating of the user. The wearable device 100 according to an embodiment of the disclosure may estimate the sweat loss, based on the selected at least one data and the user's exercise time. For example, the wearable device 100 according to an embodiment of the disclosure may provide the user with the average of a plurality of data (e.g., data having a time of sweating included within the second designated error range having an error range wider than the first designated error range) having a time of sweating similar to the time of sweating of the user as a coordinate value of the X axis, as information about the sweat loss. When the sweat loss exceeds a designated amount (e.g., 500 mL) or a designated ratio (e.g., 2% of the body weight) during the exercise of the user, the wearable device 100 according to an embodiment of the disclosure may provide a feedback for requesting water intake to the user through the display module 130 or another output device (e.g., a speaker). Alternatively, when the sweat loss exceeds a designated amount (e.g., 500 mL) or a designated ratio (e.g., 2% of body weight) after the user finishes exercise, the wearable device 100 according to an embodiment of the disclosure may provide a feedback requesting water intake to the user through the display module 130 or another output device (e.g., a speaker). The sweat loss according to an embodiment of the disclosure may be determined based on parameters included in Equation 1 below.$Sweat Loss = A \left(Ts\right) ∗ B ∗ C ∗ \left(Te-Ts\right)$

In Equation 1, A(Ts) is a sweat loss characteristic coefficient, and may be a function having a characteristic in which A(Ts) increases as Ts (e.g., time of sweating) decreases. In Equation 1, B may mean an activity intensity coefficient, and C may refer to a correction coefficient based on the basic information about the user. In Equation 1, Te may mean the time when exercise ends. An electronic device according to the related art provides sweat loss information to the user without considering A(Ts) and Te-Ts, thereby providing relatively inaccurate information. However, the wearable device 100 according to an embodiment of the disclosure may provide sweat loss information to the user considering both A(Ts) and Te-Ts, and thus providing the user with relatively accurate sweat loss information.

The wearable device 100 according to an embodiment of the disclosure may estimate the environment information about the place where the user is currently positioned, based on the time of sweating of the user and/or the selected data. For example, the wearable device 100 according to an embodiment of the disclosure may estimate the environment information about the place where the user is currently positioned by comparing the average of the times of sweating of the user for a designated past period with the current time of sweating of the user. Alternatively, the wearable device 100 according to an embodiment of the disclosure may estimate the environment information about the place where the user is currently positioned by comparing the average of the times of sweating of another user for a designated past period with the current time of sweating of the user. The wearable device 100 according to an embodiment of the disclosure may estimate that the user is currently in an environment where the temperature is high, e.g., when the average of the times of sweating of the user for one month is identified as 5 minutes and the current time of sweating of the user is identified as 2 minutes. The wearable device 100 according to an embodiment of the disclosure may determine whether the difference between the times of sweating exceeds a designated error range, e.g., when the average of the times of sweating of other users for one month is identified as 10 minutes and the current time of sweating of the user is identified as 2 minutes. The wearable device 100 according to an embodiment of the disclosure may estimate that the user is currently in an environment where the temperature is high, when the difference between the times of sweating exceeds a designated error range.

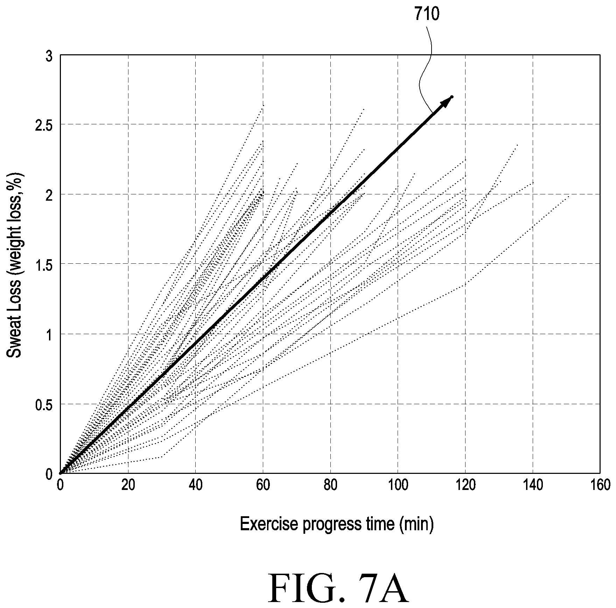

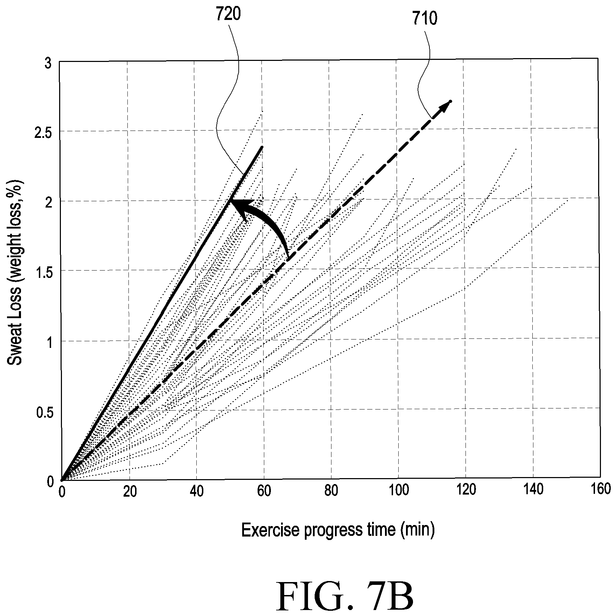

FIG. 7A illustrates a function or operation of estimating a sweat loss and providing an estimated sweat loss to a user according to the related art. FIG. 7B illustrates a function or operation of estimating a sweat loss based on a sweating characteristic of a user and providing an estimated sweat loss to a user according to an embodiment of the disclosure.

Referring to FIG. 7A, as illustrated in FIG. 7A, the electronic device according to the related art provides the user with the average of the plurality of data according to the basic information about the user as information about the estimated sweat loss without considering the user's sweating characteristics at all. For example, information about the sweat loss is provided to the user based on first data 710. However, the wearable device 100 according to an embodiment of the disclosure may provide the user with relatively accurate sweat loss information, as compared with the related art, by providing sweat loss information based on data (e.g., second data 720) customized for the user among the sample data, rather than the average of the sample data as shown in FIG. 7B, considering even the user's sweating characteristics (e.g., whether the user has a constitution of sweating a lot, whether the user has a constitution of sweating fast, or whether the user has a constitution of sweating little), as well as the user basic information. FIGS. 7A and 7B, for convenience of description, illustrates that a plurality of data having a designated slope starts from the origin (e.g., (0,0)), but may start at various positions depending on the time of sweating.

A wearable device (e.g., the wearable device 100 of FIG. 1) according to an embodiment of the disclosure may comprise at least one first sensor (e.g., the sensor module 140 of FIG. 1), at least one second sensor (e.g., the sensor module 140 of FIG. 1) including a plurality of electrodes, at least one processor (e.g., the processor 110 of FIG. 1), and memory (e.g., the memory 120 of FIG. 1) storing instructions. The instructions, when executed by the at least one processor, cause the wearable device to, identify whether, using the at least one first sensor, a user wearing the wearable device starts exercise, based on the identifying that the user starts the exercise, identify, using the at least one second sensor, a time of sweating of the user; and based on the identified time of sweating, determine a degree of sweat loss of the user.

A method for controlling a wearable device (e.g., the wearable device 100 of FIG. 1) according to an embodiment of the disclosure may comprise identifying whether, using the at least one first sensor (e.g., the sensor module 140 of FIG. 1), a user wearing the wearable device starts exercise, based on the identifying that the user starts the exercise, identifying, using the at least one second sensor (e.g., the sensor module 140 of FIG. 1), a time of sweating of the user, and based on the identified time of sweating, determining a degree of sweat loss of the user.

The electronic device according to various embodiments of the disclosure may be one of various types of electronic devices. The electronic devices may include, for example, a portable communication device (e.g., a smart phone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment of the disclosure, the module may be implemented in a form of an application-specific integrated circuit (ASIC).

Various embodiments as set forth herein may be implemented as software (e.g., the program 2540) including one or more instructions that are stored in a storage medium (e.g., internal memory 2536 or external memory 2538) that is readable by a machine (e.g., the electronic device 2501). For example, a processor of the machine (e.g., the electronic device 2501) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The storage medium readable by the machine may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment of the disclosure, a method according to various embodiments of the disclosure may be included and provided in a computer program product. The computer program products may be traded as commodities between sellers and buyers. The computer program product may be distributed in the form of a machine-readable storage medium (e.g., compact disc read only memory (CD-ROM)), or be distributed (e.g., downloaded or uploaded) online via an application store (e.g., Play Store^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to an embodiment of the disclosure, each component (e.g., a module or a program) of the above-described components may include a single entity or multiple entities. Some of the plurality of entities may be separately disposed in different components. According to an embodiment of the disclosure, one or more of the above-described components may be omitted, or one or more other components may be added. Alternatively or additionally, a plurality of components (e.g., modules or programs) may be integrated into a single component. In such a case, according to various embodiments of the disclosure, the integrated component may still perform one or more functions of each of the plurality of components in the same or similar manner as they are performed by a corresponding one of the plurality of components before the integration. According to an embodiment of the disclosure, operations performed by the module, the program, or another component may be carried out sequentially, in parallel, repeatedly, or heuristically, or one or more of the operations may be executed in a different order or omitted, or one or more other operations may be added.

It will be appreciated that various embodiments of the disclosure according to the claims and description in the specification can be realized in the form of hardware, software or a combination of hardware and software.

Any such software may be stored in non-transitory computer readable storage media. The non-transitory computer readable storage media store one or more computer programs (software modules), the one or more computer programs include computer-executable instructions that, when executed by one or more processors of an electronic device, cause the electronic device to perform a method of the disclosure.

Any such software may be stored in the form of volatile or non-volatile storage, such as, for example, a storage device like read only memory (ROM), whether erasable or rewritable or not, or in the form of memory, such as, for example, random access memory (RAM), memory chips, device or integrated circuits or on an optically or magnetically readable medium, such as, for example, a compact disk (CD), digital versatile disc (DVD), magnetic disk or magnetic tape or the like. It will be appreciated that the storage devices and storage media are various embodiments of non-transitory machine-readable storage that are suitable for storing a computer program or computer programs comprising instructions that, when executed, implement various embodiments of the disclosure. Accordingly, various embodiments provide a program comprising code for implementing apparatus or a method as claimed in any one of the claims of this specification and a non-transitory machine-readable storage storing such a program.

While the disclosure has been shown and described with reference to various embodiments thereof, it will be understood by those skilled in the art that various changes in form and details may be made therein without departing from the spirit and scope of the disclosure as defined by the appended claims and their equivalents.

## Claims

1. A wearable device comprising:
at least one first sensor;
at least one second sensor including a plurality of electrodes;
memory storing one or more computer programs; and
one or more processors communicatively coupled to the at least one first sensor, the at least one second sensor, and the memory,
wherein the one or more computer programs include computer-executable instructions that, when executed by the one or more processors individually or collectively, cause the wearable device to:
identify whether, using the at least one first sensor, a user wearing the wearable device starts exercise,
based on the identifying that the user starts the exercise, identify, using the at least one second sensor, a time of sweating of the user, and
based on the identified time of sweating, determine a degree of sweat loss of the user.

2. The wearable device of claim 1, wherein the one or more computer programs further include computer-executable instructions that, when executed by the one or more processors individually or collectively, cause the wearable device to, based on a change in impedance of the plurality of electrodes at a designated frequency, determine the time of sweating.

3. The wearable device of claim 2, wherein the one or more computer programs further include computer-executable instructions that, when executed by the one or more processors individually or collectively, cause the wearable device to, based on a change in phase value at the designated frequency, determine the time of sweating.

4. The wearable device of claim 1, wherein the one or more computer programs further include computer-executable instructions that, when executed by the one or more processors individually or collectively, cause the wearable device to select at least one data corresponding to the time of sweating among a plurality of prestored data and, based on data the selected data, determine the degree of sweat loss.

5. The wearable device of claim 1, further comprising:
a display,
wherein the one or more computer programs further include computer-executable instructions that, when executed by the one or more processors individually or collectively, cause the wearable device to provide a designated feedback through the display when the degree of sweat loss is a designated ratio or more.

6. The wearable device of claim 1, wherein the one or more computer programs further include computer-executable instructions that, when executed by the one or more processors individually or collectively, cause the wearable device to, based on the time of sweating, identify information about an ambient environment where the user is positioned.

7. The wearable device of claim 1, wherein the at least one first sensor includes at least one sensor among an acceleration sensor, a photoplethysmography (PPG) sensor, a temperature sensor, or a gyro sensor.

8. The wearable device of claim 1, wherein the one or more computer programs further include computer-executable instructions that, when executed by the one or more processors individually or collectively, cause the wearable device to, based on information about the time of sweating, identify a sweating characteristic of the user.

9. The wearable device of claim 1, wherein the plurality of electrodes are electrically connected to each other.

10. The wearable device of claim 2, wherein the designated frequency includes a frequency in a 5kHz band.

11. A method performed by a wearable device, the wearable device including at least one first sensor and at least one second sensor including a plurality of electrodes, the method comprising:
identifying, by the wearable device, whether, using the at least one first sensor, a user wearing the wearable device starts exercise;
based on the identifying that the user starts the exercise, identifying, by the wearable device, using the at least one second sensor, a time of sweating of the user; and
based on the identified time of sweating, determining, by the wearable device, a degree of sweat loss of the user.

12. The method of claim 11, further comprising:
based on a change in impedance of the plurality of electrodes at a designated frequency, determining the time of sweating.

13. The method of claim 12, further comprising:
based on a change in phase value at the designated frequency, determining the time of sweating.

14. One or more non-transitory computer-readable storage media storing one or more computer programs including computer-executable instructions that, when executed by one or more processors of a wearable device individually or collectively, the wearable device including at least one first sensor and at least one second sensor cause the wearable device to perform operations, the operations comprising:
identifying, by the wearable device, whether, using the at least one first sensor, a user wearing the wearable device starts exercise;
based on the identifying that the user starts the exercise, identifying, by the wearable device, using the at least one second sensor, a time of sweating of the user; and
based on the identified time of sweating, determining, by the wearable device, a degree of sweat loss of the user.

15. The one or more non-transitory computer-readable storage media of claim 14, the operations further comprising:
based on a change in impedance of a plurality of electrodes at a designated frequency, determining the time of sweating.
